Europäisches Patentamt

European Patent Office

Office européen des brevets

(11) Publication number: **0 301 006 B1**

## EUROPEAN PATENT SPECIFICATION

(45) Date of publication of patent specification: **06.05.92**   (51) Int. Cl.5: **A61K 9/20**, A61K 31/57

(21) Application number: **87901873.7**

(22) Date of filing: **17.02.87**

(86) International application number:
**PCT/US87/00302**

(87) International publication number:
**WO 87/05804 (08.10.87 87/22)**

(54) **METHYLPREDNISOLONE/SODIUM CARBOXYMETHYL STARCH TABLET COMPOSITION.**

(30) Priority: **01.04.86 US 846620**

(43) Date of publication of application:
**01.02.89 Bulletin 89/05**

(45) Publication of the grant of the patent:
**06.05.92 Bulletin 92/19**

(84) Designated Contracting States:
**AT BE CH DE FR GB IT LI LU NL SE**

(56) References cited:
**EP-A- 0 052 076**
**FR-A- 2 121 805**
**GB-A- 2 153 677**

**Chemical Abstracts, vol. 98, no. 2, January 1983 (Columbus, Ohio, USA), C.F. Lerk et al., "Interaction of tablet disintegrants and magnesium stearate during mixing. II. Effect on dissolution rate", see page 222, abstract no. 8124n, Pharm. Acta Helv. 1982, 57 (10-11), 282-6**

(73) Proprietor: **THE UPJOHN COMPANY**
**301 Henrietta Street**
**Kalamazoo, Michigan 49001(US)**

(72) Inventor: **LERK, Coenraad, F.**
**Braamlaan 8**
**NL-9321 GG Peize(NL)**
Inventor: **BOLHUIS, Gerad, Klaas**
**Noorderstraat 27**
**NL-9982 HB Uithuizermeeden(NL)**

(74) Representative: **Perry, Robert Edward et al**
**GILL JENNINGS & EVERY 53-64 Chancery Lane**
**London WC2A 1HN(GB)**

Chemical Abstracts, vol. 95, no. 24, December 1981 (Columbus, Ohio, USA), H. Robert et al., "Effect of the type and concentration of carboxymethylstarches on the bioavailability of aspirin in tablets", see page 368, abstract no. 209528j, C.-R.- Congr. Eur. Biopharm. Pharmacocinet., 1st. 1981, 1, 352-63

"Primojel", Avebe Product Information

## Description

Glucocorticoid steroids are marketed in many different pharmaceutical dosage forms. In tablet dosage form, the amount of steroid varies greatly. DECADRON Tablets contain 0.25, 0.50, 0.75, 1.5 mg of dexamethasone. MEDROL Tablets contain 2, 4, 8, 16, 24 or 32 mg of methylprednisolone. With prednisone, the tablets contain up to 50 mg of active ingredient. These tablets are produced by use of the usual diluents, binders, disintegration agents, lubricants and coloring agents.

It has now been found useful to administer to patients having certain medical problems such as lymphoma, leukemia, cancer, organ transplant (both pre and postoperatively) or immune complex diseases (rheumatoid arthritis, multiple sclerosis and some kidney diseases) and cerebral edema, much larger doses of glucocorticoids, in particular methylprednisolone. For example, methylprednisolone has been used in doses of up to 400 mg/m$^2$ and prednisone has been used in doses of up to 1,000 mg/m$^2$ in medical oncology. Methylprednisolone has been used in oral doses of 200-2000 mg/day to treat inoperable brain tumors. Since oral administration is preferable, it would be desirable to prepare rapidly disintegrating steroid (methylprednisolone) tablets or capsules containing much larger doses of steroid, in the range of about 50 to about 700 mg for tablets and about 50 to 1000 mg for capsules.

Further, it is desirable to have a tablet or capsule which can either be swallowed or for those individuals who can not swallow a tablet or capsule to have a tablet which will rapidly disintegrate and rapidly dissolve in an aqueous media which can then be drank by the patient. With a capsule, the contents of the capsule can be emptied into the aqueous media. If the tablet is to be produced so that it can be swallowed, it can not be an effervescent tablet. Hence, the larger tablet must be readily dispersible in an aqueous media without being effervescent.

Once a tablet or capsule has disintegrated then dissolution of the active pharmaceutical agent can take place. If one has rapid disintegration then rapid dissolution is possible. If disintegration is slow then rapid dissolution is not possible. Therefore, if one wants a rapid dissolving pharmaceutical agent one should have at least a rapid disintegrating tablet.

Most compressed tablets contain a disintegrating agent. A disintegrating agent is a substance, or mixture of substances added to the mixture of ingredients which are used to prepare a tablet, to facilitate the tablet's breakup or disintegration after administration, or disintegration when placed in water to form a solution which can be drank by the patient. The pharmaceutically active ingredient must be released from the tablet matrix as efficiently as possible to allow for its rapid dissolution. If a pharmaceutical agent is released from the tablet slowly it can not have a rapid dissolution rate. Disintegrating agents include (1) starches (corn, potato and rice; corn is the most common), (2) modified starches (sodium carboxymethyl starch also known as sodium starch glycolate), (3) microcrystalline celluloses, (4) water soluble cellulose derivatives (methyl cellulose, sodium carboxymethyl cellulose, hydroxypropyl methyl cellulose and carboxymethylcellulose calcium NF XVI), (5) crosslinked polyvinylpyrrolidones (crospovidone NF XVI), (6) water insoluble cellulose derivatives (croscarmellose sodium, Type A, NF XVI) and (7) others (Veegum HV, agar, bentonite, natural sponge, cation exchange resins, alginic acid, guar gum and citrus pulp).

Some disintegrating agents, super-disintegrants, provide for a more rapid disintegration than others. Super-disintegrants are disintegrants which can be used in a fractional amount of normal disintegrants to obtain the same effect. For example if 2-4 % of a normal disintegrant such as corn starch is used and another disintegrant gives the same effect in a fraction of a per-cent then that disintegrant would be considered a super-disintegrant. Super-disintegrants include modified starches, croscarmellose sodium, carboxymethylcellulose calcium and crospovidone. The pharmaceutical literature indicates that when compared side by side as a tablet disintegrant, with other tablet disintegrating agents, sodium starch glycolate provided the shortest disintegration time; see Pharm. Acta Helv. 44, 418 (1969). Acta Helv. 49, 248 (1974) discloses that sodium starch glycolate is a better disintegrant than others for both water-soluble and water-insoluble pharmaceutical agents. It is known that starch is much more effective as a disintegrant when used extragranularly (in the dry form) or when it is equally distributed outside (in the dry form) and inside (by wet granulation) the granules. In contrast to starch, sodium starch glycolate or other super-disintegrants (intragranular, extragranular or equally distributed) have only a small effect on tablet disintegration rate. It is also known that if too much of a water soluble cellulose derivative is used that inhibition of disintegration due to gelatinous entrapment of the tablet particles occurs. This is not true for modified starches (sodium starch glycolate), croscarmellose sodium, type A and crosprovidone when used in the common concentrations of about 4 %.

Sodium starch glycolate (sodium carboxymethyl starch) is official in the USP XX and NF XV, is marketed under the names EXPLOTAB® by Edward Mendell Co. Inc. of New York and PRIMOJEL® by Avebe Veendam of Holland Edward Mendell Co. Inc. in their product literature for EXPLOTAB® advertise it as remarkably effective for rapid disintegration and enhanced dissolution when incorporated in tablet formulations prepared by either dry, direct compression or wet granulation techniques. With regard to the amount of sodium starch glycolate to use it states, "Manufacturing experience has suggested that it is most effective in the range of 1-8% with the optimum level in the vicinity of 4%. It is recommended that the proportion to be employed in each individual formulation be determined in the laboratory." Avebe in their product literature for PRIMOJEL® state, "PRIMOJEL® should be used in concentrations of 2... 8%, calculated on the weight of the tablet. The most usual concentration is about 4%, but in many formulations a concentration of 2% will be sufficient."

Factors other than the presence of disintegrating agents can affect significantly the disintegration time of a compressed tablet. The binder, the hardness of the tablet and the lubricant all influence the disintegration time.

Rapidly dissolving tablets containing 75 to 300 mg of pharmaceutical are known. These tablets usually contain antibiotics which are in their salt form and hence quite water soluble. Tablets containing large amounts of steroid are known. For example, PROVERA Tablets contain 100 and 200 mg of medroxyprogesterone, a hydrophobic substance. Generic medroxyprogesterone tablets containing 400 and 500 mg of the pharmaceutical are known. However, neither the PROVERA Tablets nor the generic medroxyprogesterone tablets are rapidly disintegrating or dissolving.

Many pharmacologically active powders exhibit a slow dissolution rate because of a low solubility of the solid. For hydrophylic substances the dissolution can be and is generally increased by micronizing the powder (increasing its specific surface area. Most pharmacologically active substances with a low solubility are at the same time hydrophobic. Micronizing such a powder, will give agglomerates which will not deagglomerate in aqueous media, resulting in a decreased dissolution rate, because of a decreased wetted surface. Applying conventional formulations and conventional processing conditions for the formulation of tablets, a slow dissolution rate can be expected, even when the tablets disintegrate rapidly. It is known that if the surface of a hydrophobic pharmaceutical agent is coated with a hydrophylic substance the dissolution time will be decreased. A number of disintegrating agents can serve a dual purpose of being a disintegrating agent and if used to coat the hydrophobic pharmaceutical, a rapid dissolution agent as well. Coating of a hydrophobic pharmaceutical with a disintegrant is only possible when the particle size of the pharmaceutical is much larger than that of the disintegrant. For micronized pharmacologically active ingredients the disintegrant will be coated by the active ingredient.

Contrary to the literature, it has been discovered that large amounts of super-disintegrants (modified starch, croscarmellose sodium, carboxymethylcellulose calcium and crospovidone will provide rapid disintegration.

Disclosed is a compressed tablet produced by a wet massing procedure which comprises methylprednisolone and greater than 10 % of a super-disintegrant.

Further disclosed is a capsule containing methylprednisolone and greater than 10 % of a super-disintegrant.

The pharmaceutical composition of the present invention, the compressed tablet, is made following known procedures, in particular wet massing.

Water soluble cellulose derivatives such as methyl cellulose and sodium carboxymethyl cellulose are hydrophylic substances which are useful in hydrophilization of hydrophobic pharmacologically active substances. Hydrophilization is needed when a coarse hydrophobic agent is formulated into a tablet containing the conventional concentration of a disintegrant (for example 10 - 20 % starch) or super-disintegrant (2 - 8 %). However, the hydrophilization step with methylcellulose has only a limited effect on the dissolution rate of methylprednisolone as compared to the effect of the wet massing procedure when more than 10 % of a super-disintegrant is used. Therefore, it is possible to perform the moistening with water rather than an aqueous mixture of a hydrophilizing agent.

The preferred formulation and process for the 100 mg methylprednisolone tablet is found in Example 3. The sequence of the addition of the modified starch, water or the hydrophilizing solution to the pharmacologically active agent(s) is not important for the effect on disintegration of the tablets and the drug dissolution. In a preferred method, the active pharmacological agent(s) is first hydrophilized with an appropriate agent, such as methylcellulose, as is known to those skilled in the art. The super-disintegrating agent is now added. Super-disintegrants include modified starches, croscarmallose sodium, carboxymethylcellulose calcium and crospovidone. The preferred super-disintegrant is the modified starch. It is preferred that the modified starch is sodium carboxymethyl starch. The amount of super-disintegrant is > 10%. It is preferred that the super-disintegrant be present in an amount of from 10 - 60%, more preferably 20 - 35%. With methylprednisolone 100 mg tablets it is most preferred the modified starch be present in an amount of about 25 to about 30 %. Sufficient water is added until the mass is in the correct consistency for wet massing. The amount of water needed depends on the amount of microcrystalline cellulose. The mass is then screened or granulated thru an appropriate sized sieve, usually about a 6 mesh sieve. The granules are dried, at about 45 - 55° in a ventilated hot-air oven for about 12 to about 20 hr. Following drying the granules are rescreened using a 500 μm to a 1000 μm sieve, preferably about 750 to about 850 μm. The type of sieve used is determined by the amount of material to be screened, for example with small amounts a 850 μm sieve by hand is operable while with larger amounts an oscillating granulator with a 750 μm sieve is preferred. If desired a diluent, such as, can be added and the material mixed. A lubricant, preferably magnesium stearate, is the last raw material to be added to the mixture before tableting occurs. While about 0.5 to about 5 % magnesium stearate is operable, 2 or 3 % is preferred as it prevents sticking which can occur if 0.5 % is used. Alternatively, the same result may be obtained by mixing all raw materials (except lubricant) as powders and perform the wetting afterwards.

The tablets are compressed so that the compressed tablet obtained contains the correct quantity of methylprednisolone and meets all required regulatory requirements and disintegrates rapidly. The tablet can be swallowed or put into an aqueous media, allowed to disintegrate and the resulting mixture drank.

While the above formulation and process for the 100 mg methylprednisolone tablet is operable for a larger tablet such as 250 or 500 mg there are some minor changes in the preferred formula and process. The preferred formulation and process for the 250 and 500 mg methylprednisolone tablet is similar to that for the 100 mg with two following two exceptions, (1) no hydrophilizing agent, such as methylcellulose, is used and the intermediate wetting step is omitted. The preferred formulation for a 250 mg methylprednisolone tablet is set forth in EXAMPLE 6.

The capsules of the present invention can be filled with the same granulation as used to make the compressed tablets. It is necessary to have methylprednisolone and > 10 % of the super-disintegrant. It is preferable to also have a lubricant such as magnesium stearate. It is well known to those skilled in the art how to prepare pharmaceutical compositions for filling capsules. The capsules will hold up to 1000 mg of methylprednisolone. These capsules containing methylprednisolone and > 10 % of super-disintegrant give a rapid disintegration and fast drug release. They can either be swallowed or emptied into aqueous media, allowed to disintegrate and the resulting mixture drank.

## DEFINITIONS

The definitions and explanations below are for the terms as used throughout the entire patent application including both the specification and the claims.

Methylprednisolone refers to 11β,17α,21-trihydroxy-6a-methylpregna-1,4-diene-3,20-dione.

Sodium carboxymethyl starch refers to sodium starch glycolate.

All temperatures are in degrees Centigrade.

USP refers to the United States Pharmacopeia.

NF refers to the National Formulary (of the United States).

EXPLOTAB® refers to sodium starch glycolate marketed by Edward Mendell Co. Inc. of New York, USA.

PRIMOJEL® refers to sodium starch glycolate marketed by Avebe Veendam of Foxhol, Holland.

Sodium starch glycolate is sodium carboxymethyl starch.

Super-disintegrants are disintegrants which can be used in a fractional amount of normal disintegrants to obtain the same effect.

Pharmaceutically acceptable refers to those properties and/or substances which are acceptable to the patient from a pharmacological/toxicological point of view and to the manufacturing pharmaceutical chemist from a physical-chemical point of view regarding composition, formulation, stability, patient acceptance and bioavailability.

When solvent pairs are used, the ratios of solvents used are volume/volume (v/v).

When the percentage (%) of solid materials is referred to it means on a w/w basis.

## EXAMPLES

EXAMPLE 1 Methylprednisolone granulation

| | |
|---|---|
| Methylprednisolone USP | 46.1 % |
| Microcrystalline cellulose | 21.0 % |
| Sodium carboxymethyl starch | 32.9 % |

The methylprednisolone is hydrophilized with 1.0 % methylcellulose solution (2 gm of methylprednisolone and 1 ml of methylcellulose solution). Three times the amount of methylcellulose solution of water is added to make the mass suitable for granulating.

EXAMPLE 2 Methylprednisolone tableting composition

| | |
|---|---|
| Granulate (EXAMPLE 1) | 91.2 % |
| Lactose 100 mesh | 8.3 % |
| Magnesium stearate | 0.5 % |

The granulate of EXAMPLE 1, magnesium stearate, and lactose are mixed for 5 min and then tableted to form compressed tablets containing 100 mg methylprednisolone, meet all regulatory requirements and have the following composition:

| | |
|---|---|
| Methylprednisolone USP | 100.0 mg |
| Microcrystalline cellulose | 45.78 mg |
| Sodium carboxymethyl starch | 71.72 mg |
| Methylcellulose | 0.5 mg |
| Magnesium stearate | 1.095 mg |
| Lactose 100 mesh | 43.65 mg |
| Tablet weight | 262.745 mg |

EXAMPLE 3 10,000 Methylprednisolone 100 mg CT

| | |
|---|---|
| Methylprednisolone USP micronized | 1,000. g |
| FD and C Blue No 2 Aluminum Lake | 1.20 g |
| Methylcellulose USP 15 CPS micronized | 5. g |
| Purified water EP-USP | 500. g |
| Sodium starch glycolate | 714. g |
| Microcrystalline cellulose NF medium po. | 456. g |
| Magnesium stearate EP-NF po. food grade | 11. g |
| Purified water EP-USP    q.s. | |
| CAUTION: 1) Avoid excessive heat and moisture | |
| 2) Avoid excessive contact with methylprednisolone | |

Put the methylprednisolone and the FD and C Blue Nr 2 Aluminum Lake thru a 20 mesh screen, and mix to obtain a sufficiently dispersed color mixture.

Dissolve the methylcellulose in purified water (500 g), add this solution to the above mixture and mix until a proper wetting is obtained.

Mix the microcrystalline cellulose and sodium starch glycolate and put thru a 20 mesh screen, add to the steroid mixture and mix 5 min.

Add sufficient purified water (about 1,000 ml) to bring to a proper wetness. The wet mass is then processed by hand thru a 6 mesh screen and then air dried at 50° for 16 hr.

The dried granules are then processed thru a Fitzmill with sieve nr 1A.

Magnesium stearate is then processed thru a 20 mesh screen, added to the granulated material and mixed for 3 min.

The 2,187 kg mixture is tableted using a 9 mm, rounded, half oval punch with a cross score to give 10,000 tablets with an average weight of 218.7 mg containing 100 mg of methylprednisolone which meets all regulatory requirements.

The tablets should be stored in suitable containers, such as double plastic bags with 15 humicaps.

EXAMPLE 4 250 mg Methylprednisolone CT

Following the general procedure of EXAMPLE 3, and making non-critical variations but making tablets averaging 546.75 mg each, 4,000 compressed tablets are produced each containing 250 mg of methylprednisolone.

EXAMPLE 5 500 mg Methylprednisolone

Following the general procedure of EXAMPLE 3, and making non-critical variations, and using 5 kg methylprednisolone, 10,000 compressed tablets are produced each comprising 500 mg of methylprednisolone.

EXAMPLE 6 250 Methylprednisolone CT

| Item | % | Weight in/of final tablet |
|---|---|---|
| Methylprednisolone | 57.95 | 256.1 mg |
| FD & C Blue Nr 2 Al Lake | 0.055 | 0.24 mg |
| Sodium Starch Glycolate | 20.5 | 90.6 mg |
| Microcrystalline Cellulose | 19.5 | 86.2 mg |
| Magnesium stearate | 2.0 | 8.9 mg |
| Tablet weight | | 442 mg |

Following the general procedure of EXAMPLE 3, and making non-critical variations including omitting the intermediate wetting step, mix all the powders, then granulate with water and compress into tablets.

**Claims**

**Claims for the following Contracting States : BE, CH, DE, FR, GB, IT, LU, NL, SE**

1. A compressed tablet which comprises, or a capsule which contains, a composition, producible by a wet-massing procedure, of methylprednisolone and greater than 10% by weight of a super-disintegrant.

2. A tablet according to claim 1, which comprises 50 to 700 mg methylprednisolone.

3. A tablet according to claim 2, which comprises 100 to 500 mg methylprednisolone.

4. A capsule according to claim 1, which comprises 50 to 1000 mg methylprednisolone.

5. A tablet or capsule according to any preceding claim, wherein the super-disintegrant is selected from modified starches, croscarmallose sodium, carboxymethylcellulose calcium and crospovidone.

6. A tablet or capsule according to claim 5, wherein the super-disintegrant is a modified starch.

7. A tablet or capsule according to claim 6, wherein the modified starch is sodium carboxymethyl starch.

**8.** A tablet or capsule according to claim 6 or claim 7, wherein the modified starch is present in an amount of from 10 to 60% by weight.

**9.** A tablet or capsule according to claim 8, wherein the modified starch is present in an amount of from 20 to 35% by weight.

**10.** A tablet or capsule according to any preceding claim, wherein the methylprednisolone is micronised.

**Claims for the following Contracting State : AT**

**1.** A method for preparing a compressed tablet which comprises, or a capsule which contains, a composition of methylprednisolone and greater than 10% by weight of a super-disintegrant, the method comprising wet-massing the methylprednisolone and the super-disintegrant.

**2.** A method according to claim 1, for the production of a tablet which comprises 50 to 700 mg methylprednisolone.

**3.** A method according to claim 1, for the production of a tablet which comprises 100 to 500 mg methylprednisolone.

**4.** A method according to claim 1, for the production of a capsule which comprises 50 to 1000 mg methylprednisolone.

**5.** A method according to any preceding claim, wherein the super-disintegrant is selected from modified starches, croscarmallose sodium, carboxymethylcellulose calcium and crospovidone.

**6.** A method according to claim 5, wherein the super-disintegrant is a modified starch.

**7.** A method according to claim 6, wherein the modified starch is sodium carboxymethyl starch.

**8.** A method according to claim 6 or claim 7, wherein the modified starch is present in an amount of from 10 to 60% by weight.

**9.** A method according to claim 8, wherein the modified starch is present in an amount of from 20 to 35% by weight.

**10.** A method according to any preceding claim, wherein the methylprednisolone is micronised.

**Revendications**

**Revendications pour les Etats contractants suivants : BE, CE, DE, FR, GB, IT, LU, NL, SE**

**1.** Comprimé produit par compression qui comprend, ou bien gélule qui contient, une composition, pouvant être produite par un procédé d'agglomération par voie humide, de méthylprednisolone et d'une quantité supérieure à 10 % en poids d'un agent de délitement hautement efficace.

**2.** Comprimé suivant la revendication 1, qui comprend 50 à 700 mg de méthylprednisolone.

**3.** Comprimé suivant la revendication 2, qui comprend 100 à 500 mg de méthylprednisolone.

**4.** Gélule suivant la revendication 1, qui comprend 50 à 1000 mg de méthylprednisolone.

**5.** Comprimé ou gélule suivant l'une quelconque des revendications précédentes, dans lequel l'agent de délitement hautement efficace est choisi entre des amidons modifiés, le croscarmellose sodique, la carboxyméthylcellulose calcique et la crospovidone.

**6.** Comprimé ou gélule suivant la revendication 5, dans lequel l'agent de délitement hautement efficace consiste en un amidon modifié.

**7.** Comprimé ou gélule suivant la revendication 6, dans lequel l'amidon modifié consiste en carboxyméthylamidon sodique.

**8.** Comprimé ou gélule suivant la revendication 6 ou la revendication 7, dans lequel l'amidon modifié est présent en une quantité de 10 à 60 % en poids.

**9.** Comprimé ou gélule suivant la revendication 8, dans lequel l'amidon modifié est présent en une quantité de 20 à 35 % en poids.

**10.** Comprimé ou gélule suivant l'une quelconque des revendications précédentes, dans lequel la méthylprednisolone est micronisée.

**Revendications pour l'Etat contractant suivant : AT**

**1.** Procédé de préparation d'un comprimé formé par compression qui comprend, ou bien d'une gélule qui contient, une composition de méthylprednisolone et d'une quantité supérieure à 10 % en poids d'un agent de délitement hautement efficace, procédé consistant à soumettre la méthylprednisolone et l'agent de délitement hautement efficace à une agglomération par voie humide.

**2.** Procédé suivant la revendication 1, pour la production d'un comprimé qui comprend 50 à 700 mg de méthylprednisolone.

**3.** Procédé suivant la revendication 1, pour la production d'un comprimé qui comprend 100 à 500 mg de méthylprednisolone.

**4.** Procédé suivant la revendication 1, pour la production d'une gélule qui comprend 50 à 1000 mg de méthylprednisolone.

**5.** Procédé suivant l'une quelconque des revendications précédentes, dans lequel l'agent de délitement hautement efficace est choisi entre des amidons modifiés, le croscarmellose sodique, la carboxyméthylcellulose calcique et la crospovidone.

**6.** Procédé suivant la revendication 5, dans lequel l'agent de délitement hautement efficace consiste en un amidon modifié.

**7.** Procédé suivant la revendication 6, dans lequel l'amidon modifié consiste en carboxyméthylamidon sodique.

**8.** Procédé suivant la revendication 6 ou 7, dans lequel l'amidon modifié est présent en une quantité de 10 à 60 % en poids.

**9.** Procédé suivant la revendication 8, dans lequel l'amidon modifié est présent en une quantité de 20 à 35 % en poids.

**10.** Procédé suivant l'une quelconque des revendications précédentes, dans lequel la méthylprednisolone est micronisée.

**Patentansprüche**

**Patentansprüche für folgende Vertragsstaaten : BE, CH, DE, FR, GB, IT, LU, NL, SE**

**1.** Komprimierte Methylprednisolon-Tablette oder -Kapsel, welche mit einem naß-verarbeitenden Herstellungsverfahren herstellbar ist und welche mehr als 10 Gew.-% eines Superdisintegrationsmitteis enthält.

**2.** Tablette nach Anspruch 1, welche 50 bis 700 mg Methylprednisolon enthält.

**3.** Tablette nach Anspruch 2, welche 100 bis 500 mg Methylprednisolon enthält.

4. Kapsel nach Anspruch 1, welche 50 bis 1000 mg Methylprednisolon enthält.

5. Tablette oder Kapsel nach einem der vorhergehenden Ansprüche, worin das Superdisintegrationsmittel aus modifizierten Stärken, Natriumcroscarmallose, Calziumcarboxymethylzellulose und Crospovidon gewählt ist.

6. Tablette oder Kapsel nach Anspruch 5, worin das Superdisintegrationsmittel eine modifizierte Stärke ist.

7. Tablette oder Kapsel nach Anspruch 6, worin die modifizierte Stärke Natriumcarboxymethylstärke ist.

8. Tablette oder Kapsel nach Anspruch 6 oder 7, worin die modifizierte Stärke in einer Menge von 10 bis 60 Gew.-% vorliegt.

9. Tablette oder Kapsel nach Anspruch 8, worin die modifizierte Stärke in einer Menge von 20 bis 35 Gew.-% vorliegt.

10. Tablette oder Kapsel nach einem der vorhergehenden Ansprüche, worin das Methylprednisolon mikronisiert ist.

**Patentansprüche für folgende Vertragsstaaten : AT**

1. Verfahren zur Herstellung einer komprimierten Tablette oder einer Kapsel, welche eine Zusammensetzung von Methylprednisolon und mehr als 10 Gew.-% eines Superdisintegrationsmittels enthält, welches Verfahren das Naß-verarbeiten von Methylprednisolon und dem Superdisintegrationsmittel umfaßt.

2. Verfahren nach Anspruch 1, zur Herstellung einer Tablette, welche 50 bis 700 mg Methylprednisolon enthält.

3. Verfahren nach Anspruch 1, zur Herstellung einer Tablette, welche 100 bis 500 mg Methylprednisolon enthält.

4. Verfahren nach Anspruch 1, zur Herstellung einer Kapsel, welche 50 bis 1000 mg Methylprednisolon enthält.

5. Verfahren nach einem der vorhergehenden Ansprüche, worin das Superdisintegrationsmittel aus modifizierten Stärken, Natriumcroscarmallose, Calziumcarboxymethylzellulose und Crospovidon gewählt ist.

6. Verfahren nach Anspruch 5, worin das Superdisintregrationsmittel eine modifizierte Stärke ist.

7. Verfahren nach Anspruch 6, worin die modifizierte Stärke Natriumcarboxymethylstärke ist.

8. Verfahren nach Anspruch 6 oder 7, worin die modifizierte Stärke in einer Menge von 10 bis 60 Gew.-% vorliegt.

9. Verfahren nach Anspruch 8, worin die modifizierte Stärke in einer Menge von 20 bis 35 Gew.-% vorliegt.

10. Verfahren nach einem der vorhergehenden Ansprüche, worin das Methylprednisolon mikronisiert ist.